# EUROPÄISCHE PATENTSCHRIFT

(11) **EP 1 349 895 B1**
(45) Veröffentlichungstag und Bekanntmachung des Hinweises auf die Patenterteilung: **09.09.2009**
(21) Anmeldenummer: 02703541.9
(22) Anmeldetag: 09.01.2002
(51) Int. Cl.: C08L 83/04, A61K 6/10

(54) **TRENNMITTEL**
SEPARATING AGENT
AGENT DE SEPARATION

(30) Priorität: 10.01.2001 DE 10100736
(43) Veröffentlichungstag der Anmeldung: 08.10.2003
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 35745 Herborn (DE); SCHLEICHER, Wolfgang, 93339 Riedenburg (DE)
(74) Vertreter: KEIL & SCHAAFHAUSEN Patentanwälte
(86) Internationale Anmeldenummer: PCT/EP2002/000136
(87) Internationale Veröffentlichungsnummer: WO 2002/055605

(56) Entgegenhaltungen:
- DE-A- 2 919 811
- US-A- 5 270 110

## Beschreibung

Die folgende Erfindung betrifft ein Trennmittel in Form eines Trennsprays mit oder ohne Treibgas und einer weiteren Komponente oder in Form eines in Lösungsmittel gelösten Trennmittels zum Aufpinseln oder Eintauchen und einer weiteren Komponente sowie ein Verfahren zur Herstellung eines positiven Abdrucks eines Gegenstandes von mit Silikonen abgeformten Gegenständen.

Beim Abformen von Gegenständen wird zunächst meist mit einer elastomeren Abformmasse ein Negativabdruck erzeugt, der dann durch Ausgießen mit einem weiteren Abformmaterial in ein positives Gegenstück des abzuformenden Gegenstand überführt wird. Dabei werden Abformmaterialien eingesetzt, die von einem flüssigen bis zähflüssigen oder plastischen Zustand in einen formstabilen Zustand übergehen können. Organische und/oder anorganische Materialien kommen zum Einsatz. Zu den erstgenannten gehören insbesondere Silikone, die durch chemische Reaktionen nach erfolgtem Abdruck aushärten und somit den abzuformenden Gegenstand in einer negativen Form wiedergeben. Als anorganisches Material, das zur Abformung eingesetzt wird, dient insbesondere Gips.

In der Dentaltechnik kommt es regelmäßig auf sehr exakte Abdrucknahme an. So werden zum Beispiel bei der Herstellung von Inlays, Onlays oder Brücken die vom Zahnarzt präparierten Zähne durch Abdrucknahme und Dublieren abgeformt.

Die Abdrucknahme erfolgt dabei üblicherweise mit Hilfe von Silikonen, die nach Verbringen in den Mund, Aushärten und nach Entnahme, ggf. gefolgt von weiteren Verarbeitungsmaßnahmen, ein negatives Modell der Präparation ergeben. Dieses Präparationsmodell muss dann vom Zahntechniker mittels weiterer Abdrucknahme in ein Arbeitsmodell überführt werden. Dazu wird üblicherweise Gips verwendet.

Die Abdrucknahme d.h. das Ausgießen des Negativmodells mit additionsvernetzendem Silikon scheidet dann aus, wenn das Negativmodell selbst aus den üblicherweise vom Zahnarzt verwendeten additionsvernetzendem Silikon hergestellt wurde. Es wäre aber von Vorteil, wenn der Zahnarzt direkt stuhlseitig den zweiten Abdruck d.h. das Ausgießen zur Herstellung eines Arbeitsmodells vornehmen könnte, unter Verwendung der additionsvernetzenden Silikone, z.B. eines extra harten Modellsilikons. Dies ist aber nicht möglich, da der Negativabdruck aus additionsvernetzenden Silikonen bei Abdrucknahme mit additionsvernetzenden Silikonen mit dem Gegenabdruck untrennbar verbunden wird.

Nach dem Stand der Technik sind sogenannte Trennmittel bekannt, die nach Erstellen des ersten Abdruckes auf die Abform aufgetragen werden. Sie sollen beim Erstellen des zweiten Abdruckes von diesem ersten Negativabdruck verhindern, dass das zweite Abdruckmaterial an das erste bindet.

Es ist ein Isolant von DeTrey zum Isolieren von Gips gegen Gips und Gips gegen Kunststoff bekannt. Zum Isolieren von Modellen und Modellstümpfen wird eine Siliconisolierung der Fa. Hedent verwendet. Beide Trennmittel sind zur Isolierung von additionsvernetzenden Siliconen gegen additionsvernetzende Silicone ungeeignet.

Ein weiteres Trennmittel ist das Silikon-Spray der Fa. Orbis Dental. Dieses ist ein Universaltrennspray auf der Basis von Trimethylsiloxy endgestopptem Polydimethylsiloxan (nicht funktionelle Silicone) und separiert Gips gegen Gips (Stumpfmodell), Gips gegen Kunststoff (Stumpfmodellerstellung), Gips gegen Gummi (Modellsockel), Gips gegen Metall (Artikulator), Gips gegen Wachs und Kunststoff gegen Kunststoff (Modellguß).

Obwohl dieses Trennmittel zur Isolierung von additionsvernetzenden Siliconen gegen additionsvernetzende Silicone eher geeignet ist als die o.g. Trennmittel, kann es dennoch keine ausreichende Trennwirkung zwischen additionsvernetzenden Materialien bewirken.

Das der Erfindung zu Grunde liegende technische Problem ist es somit, eine Möglichkeit zu schaffen, mit der es gelingt aus additionsvernetzenden Silikonen hergestellte Abdrücke mit additionsvernetzenden Silikonen, insbesondere mit besonders harten Modellsilikonen abzuformen.

Das Problem wird gelöst durch ein Trennmittel in Form eines Trennsprays mit oder ohne Treibgas und einer weiteren Komponente oder in Form eines in Lösungsmittel gelösten Trennmittels zum Aufpinseln oder Eintauchen und einer weiteren Komponente, ausgewählt aus der Gruppe umfassend Silikone, Silikonharze, Silane, Silandendrimere und Tenside, die pro Molekül eine einzige ethylenisch ungesättigte Struktureinheit aufweisen, bzw. statistische Gemische von Molekülen/Polymeren, die pro Molekül im Durchschnitt eine einzige ethylenisch ungesättigte Struktureinheit aufweisen. Vorzugsweise weist das erfindungsgemäße Trennmittel als ethylenisch ungesättigte Struktureinheit eine Vinylgruppe auf, die direkt oder über einen Linker (Spacer) am Si-Atom gebunden ist.

Im erfindungsgemäßen Trennmittel können die folgenden Verbindungen enthalten sein:
1) Silane, die pro Molekül eine ethylenisch ungesättigte Struktureinheit aufweisen, wie Alkenylalkylsilane, Alkenylarylsilane, Alkenylalkylarylsilane, Alkenyl(alkylsiloxy)silane, Alkenyl(arylsiloxy)-silane, Alkenyl(alkylarylsiloxy)silane, wobei die Alkyl- und/oder Arylgruppen auch weitere Substituenten enthalten können. Insbesondere werden Silane aus der Gruppe Vinyltrimethylsilan, Vinyltriethylsilan, Vinyldiethylmethylsilan, Vinyl-t-butyldimethylsilan, Vinylphenyldimethylsilan, Vinyl(trifluoromethyl)dimethylsilan, Vinyl-(3,3,3-trifluorpropyl)dimethylsilan, Vinyltriphenylsilan und Vinyltris-(trimethylsiloxy)silan eingesetzt. Diese Silane können auch Bestandteil von Gemischen sein, wie sie bei der technischen Synthese der Silane entstehen. Das bedeutet, dass die Reinheit der entsprechenden Silane zwischen 50 und 100 %, insbesondere 75 und 100 % liegt. Die Silane mit ungesättigter ethylenischer Struktureinheit werden bezogen auf die Gesamtmasse des Trennmittels in einem Gewichtsbereich von 0,01 bis 50 Gew. %, insbesondere 0,1 bis 20 Gew. % eingesetzt.
2) Silikonpolymere, die pro Molekül eine ethylenisch ungesättigte Struktureinheit aufweisen, wie α-Alkenyl,-ω-trimethylsiloxy endgestoppte Polydialkylsiloxane, Polydiarylsiloxane und Polyalkylarylsiloxane, wobei die Alkyl- und/oder Arylgruppen gegebenenfalls auch weitere Substituenten enthalten können. Insbesondere werden α-Vinyl-,ω-trimethylsiloxy-Polydimethylsiloxane der allgemeinen Formel 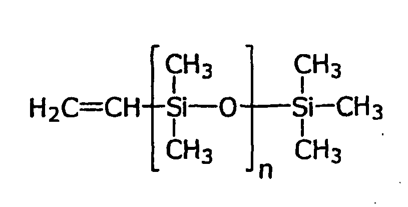 mit n = 1 bis 6000 eingesetzt.
   Diese Silikonpolymere können auch Bestandteil statistischer Gemische sein, wie sie nach dem Stand der Technik durch Äquilibrierung und Kondensation erhalten werden (siehe Walter Noll in "Chemie und Technologie der Silicone", Verlag Chemie GmbH, Weinheim/Bergstr, 2. Auflage 1968, S. 179 ff.) Beispielsweise kann in dem erfindungsgemäßen Trennmittel ein Gemisch bestehend aus
   25 % 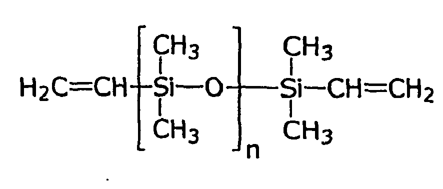 mit n = 1 bis 6000,
   25 % 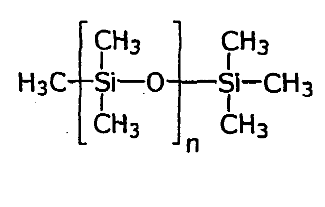 mit n = 1 bis 6000
   und 50 % 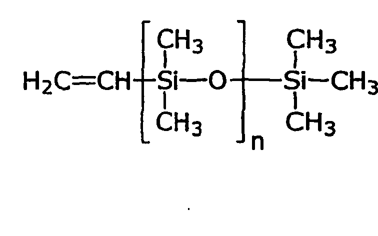 mit n = 1 bis 6000 eingesetzt werden.
   Die Silikonpolymere mit einer ethylenisch ungesättigten Struktureinheit werden bezogen auf die Gesamtmasse des Trennmittels in einem Gewichtsbereich von 0,01 bis 50 Gew.%, insbesondere 0,1 bis 20 Gew.% eingesetzt.
3) Zyklische Oligo- oder Polydialkyl-, -diaryl- oder -alkylaryl-siloxane, die pro Molekül eine ethylenisch ungesättigte Struktureinheit aufweisen.
   Insbesondere werden zyklische Vinyloligo- oder Vinylpolydimethylsiloxane wie beispielsweise Vinylheptamethyl-zyklotetrasiloxan eingesetzt.
   Diese zyklischen Siloxane können auch in Form eines durch Äquilibrierung oder Kondensation hergestellten statistischen Gemisches eingesetzt werden. Die zyklischen Siloxane mit einer ethylenisch ungesättigten Struktureinheit werden bezogen auf die Gesamtmasse des Trennmittels in einem Gewichtsbereich von 0,01 bis 50 Gew.%, insbesondere 0,1 bis 20 Gew.% eingesetzt.
4) Feste oder flüssige QM-Harze (gemäß Walter Noll, "Chemie und Technologie der Silicone", Verlag Chemie GmbH Weinheim, Auflage 1968, S. 3 ), die pro Molekül eine ethylenisch ungesättigte Struktureinheit aufweisen. Diese QM-Harze enthalten als Q-Einheiten das tetrafunktionelle SiO_{4/2}, als M-Baustein das monofunktionelle R₃ SiO_{½}, als M-Baustein das monofunktionelle R₃ SiO_{½}, als T-Einheiten das trifunktionelle R SiO_{3/2} und als D-Einheiten das bifunktionelle R₂ SiO_{2/2}, wobei R = Vinyl-, Methyl-, Ethyl- und Phenyl sein kann.
   Die Silikonharze können außer den Resten R noch kleine Anteile an Hydroxyl- oder Alkoxygruppen enthalten, die an die Si-Atome gebunden sind. Die QM-Harze mit einer ethylenisch ungesättigten Struktureinheit können in Form eines statistischen Gemisches, das bei deren technischen Herstellung entsteht, eingesetzt werden. Die genannten Silikonharze können auch in organischen Lösungsmitteln gelöst vorliegen. Die QM-Harze werden bezogen auf die Gesamtmasse des Trennmittels in einem Gewichtsbereich von 0,01 bis 50%, insbesondere 0,1 bis 20 Gew.%, eingesetzt.
5) Silandendrimere oder Siloxandendrimere, die pro Molekül eine terminale Alkenylgruppe aufweisen. Silandendrimere weisen die allgemeine Formel auf:
   Si R¹ ₓ [(CH₂)ₙ - Si- (CH₂)ₘ - CH = CH₂]₁ [(CH₂)ₙ - Si- (CH₂)ₘ - CH₃] ₃₋ₓ
   mit R¹ = Alkyl, Aryl, vorzugsweise Methyl, Phenyl
   mit n = 2 bis 5
   mit m = 0 bis 3
   mit x = 0 bis 3

   Siloxandendrimere weisen die allgemeine Formel auf:
   R¹₆₋ₘ Si R²ₘ - O - (SiR²₂-O)ₙ-SiR²ₘ R¹₆₋ₘ
   mit R¹ = siehe oben
   mit R² = Alkenyl, vorzugsweise Allyl, Vinyl
   mit m = 4 bis 6
   mit n = 0 bis 5

   Insbesondere werden eingesetzt:

   H₃C - Si [(CH₂ - CH₂ - Si (CH₃)₃]₂ (CH₂ - CH₂ - SiR₂ - CH = CH₂),

   Si [CH₂ - CH₂ - Si(CH₃)₂ - CH = CH₂] [CH₂ - CH₂ - Si (CH₃)₃]₃

   oder 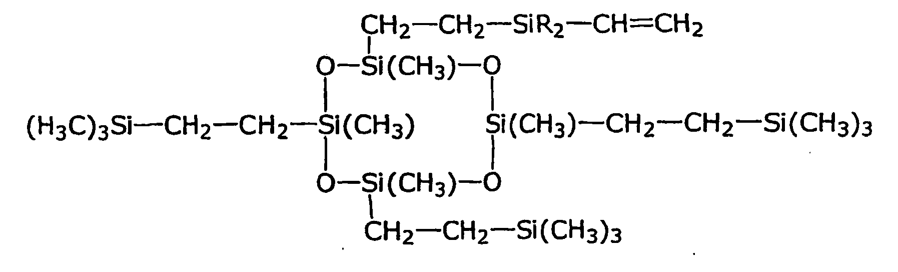
6) Tenside, die eine Alkenyl- oder Si-Alkenyl-Struktureinheit enthalten. Es handelt sich um anionische Tenside, insbesondere solche Derivate der Alkylsulfate, Alkylbenzolsulfonate und- phosphate, kationischen Tenside, insbesondere Tetralkylammoniumhalogenide, -sulfate oder - acetate oder Alkylpyridiniumhalogenide, nicht ionischen Tenside, insbesondere Alkyl- und Alkylaryl - Polyethylenglycole und -glycolether; Fettsäurealkylolamide, Saccharosefettsäureester, Trialkylamid, Silikontenside oder Fluortenside sowie amphoteren Tenside, insbesondere sulfatierten oder oxyethylierten Kondensationsprodukte aus Alkylphenolen und Formaldehyd, Ethylenoxid-Propylenoxid-Blockpolymerisate, modifizierten Polysiloxane, Polyvinyl N-alkylpyridiniumsalze oder Copolymerisate aus Vinylpyridin und Methacrylsäure, bei denen eine Alkylgruppe durch eine Alkenyl- oder Si-Alkenyl Struktureinheit, insbesondere eine Vinyl- und Si-Vinyl-Gruppe, substituiert ist.
7) Sillkonpolymere ohne funktionelle Struktureinheiten, wie α,ω-Bis(trialkylsiloxy) endgestoppte Polydialkylsiloxane, α,ω-Bis(triarylsiloxy) endgestoppte Polydiarylsiloxane und α,ω-Bis(alkylarylsiloxy) endgestoppte Polyalkylarylsiloxane. Insbesondere werden α,ω-Bis(trimethylsiloxy) endgestoppte Polydimethylsiloxane der allgemeinen Formel 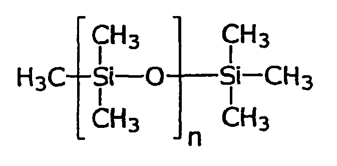 mit n = 1 bis 6000 eingesetzt.

Das erfindungsgemäße Trennmittel in Form eines Trennsprays mit Treibgas enthält als Treibgas typischerweise Propan und/oder Butan.

Das erfindungsgemäße Trennmittel in gelöster Form zum Eintauchen oder Auftragen enthält als Lösungsmittel typischerweise organische Lösungsmittel wie Aromaten, Benzine, Ether, Ester Alkohole, Ketone, Aldehyde, Amine, Carbonsäuren, halogenierte Lösungsmittel, z.B. Chloroform, zyklische oder lineare Oligo- oder Polysiloxane oder Wasser oder mögliche Kombinationen aus den oben genannten Lösungsmitteln.

Neben dem Treibgas bzw. Lösungsmittel enthält das erfindungsgemäße Trennmittel oberflächenaktive Verbindungen, die nach Möglichkeit während des Austretens aus einem das Trennmittel enthaltenden Behälter mit den anderen Inhaltsstoffen keinen Schaum bilden.

Als oberflächenaktive Verbindungen werden im erfindungsgemäßen Trennmittel insbesondere anionische, kationische und/oder nicht ionische Tenside eingesetzt.

Als weitere Inhaltsstoffe können dem erfindungsgemäßen Trennmittel Mattierungsmittel und Färbemittel zugesetzt werden. Die Färbemittel werden der Trennmittelzusammensetzung zugesetzt um dem Anwender eine Einschätzung zu geben, ob und in welcher Schichtdicke und Gleichmäßigkeit er das Trennmittel auf das. zu behandelnde Substrat aufgebracht hat. Der Anwender kann am Vorhandensein und Intensität der Einfärbung auf dem Substrat nach Aufbringen des Trennmittels besser erkennen, ob weiteres Trennmittel aufgebracht werden muss, um eine optimale Trennwirkung zu erhalten (=> visuelle Kontrolle).

Bei den genannten Färbemitteln handelt es sich um lösliche Farbstoffe oder um Pigmentfarbstoffe. Zur Verwendung gelangen gleichfalls Farbpasten aus Polysiloxan- sowie Mineralöl-Farbstoff-Formulierungen.

Die Mattierungsmittel werden eingesetzt, um eine optische Abtastbarkeit oder Scanbarkeit des Modells mit einer Kamera oder einem Scanner zu erzielen. Durch das Mattierungsmittel wird ein Glänzen des Modells und somit störende Reflexe beim optischen Abtasten unterdrückt. Bei der Behandlung einer Abformung mit einem Trennmittel, das ein Mattierungsmittel enthält, befindet sich das Mattierungsmittel zunächst auf der Abformung. Nach Einbringen und Entnahme des Modellmaterials aus der Abformung verbleibt das Mattierungsmittel auf dem ausgehärteten Modellmaterial. Das Modell ist nun mit einer gut haftenden Schicht aus Mattierungsmittel überzogen und einer optischen Abtastung zugänglich.

Als Mattierungsmittel werden Metalloxide oder Metalle eingesetzt, insbesondere kommen Titanoxid, Zinkoxid und Siliciumdioxid zum Einsatz.

Der Mattierungsgrad hängt ab von der Korngröße der jeweiligen Mattierungsmittel.

Erfindungsgemäß beansprucht werden auch Verfahren zur Herstellung eines positiven Abdrucks eines Gegenstandes von mit Silikonen abgeformten Gegenständen umfassend folgende Schritte:
Abformnahme eines abzuformenden Gegenstandes mit additionsvernetzenden Silikonen,
Aushärten des additionsvernetzenden Silikons am abzuformenden Gegenstand,
Ablösen der gebildeten negativen Abformung des abzuformenden Gegenstandes aus ausgehärtetem additionsvernetzendem Silikon-Polymer,
Besprühen, Bepinseln oder Eintauchen der negativen Abformung des abzuformenden Gegenstandes mit dem bzw. in dem erfindungsgemäßen
Trennmittel, gegebenenfalls gefolgt durch eine visuelle Kontrolle, optisches Abtasten oder Scannen,
Abformen der negativen Abformung des abzuformenden Gegenstandes mit dem additionsvernetzendem Silikon, insbesondere mit extra hartem Modellsilikon und Aushärten des Modell- additionsvernetzenden Silikons,
Entfernen der positiven Abformung aus der negativen Abformung des abzuformenden Gegenstandes (positiver Abdruck des abzuformenden Gegenstandes),
ggf. gefolgt von einer weiteren Bearbeitung des positiven Abdrucks.

Die Erfindung betrifft auch ein Verfahren, bei dem der positive Abdruck des abzuformenden Gegenstandes mit einem Sockel verbunden wird, umfassend folgende Schritte:
Abformnahme eines abzuformenden Gegenstandes mit additionsvernetzenden Silikonen,
Aushärten des additionsvernetzenden Silikons am abzuformenden Gegenstand,
Ablösen der gebildeten negativen Abformung des abzuformenden Gegenstandes aus ausgehärtetem additionsvernetzendem Silikon-Polymer,
Besprühen, Bepinseln, Eintauchen der negativen Abformung des abzuformenden Gegenstandes mit dem bzw. in dem erfindungsgemäßen Trennmittel, gegebenenfalls gefolgt durch eine visuelle Kontrolle, optisches Abtasten oder Scannen,
Abformen der negativen Abformung des abzuformenden Gegenstandes mit dem additionsvernetzenden Silikon, insbesondere mit extra hartem Modellsilikon,
Aufbringen eines Sockelmaterials (standfestes mittel- bis zähfließendes additionsvernetzendes Silikon) während des Aushärtens des additionsvernetzendem Silikonmodellmaterials, um das positive Modell mit einem Sockel zu versehen und diesem den notwendigen mechanischen Halt zu geben,
Aushärten des additionsvernetzendem Silikons (extra hartes Modellsilikon) und des additionsvernetzendem Silikonsockelmaterials,
Entfernen der positiven Abformung mitsamt des fest verbundenen Sockels aus der negativen Abformung des abzuformenden Gegenstandes,
Bearbeitung des positiven Modells mit einem Skalpell, einem Messer oder einer Säge zur Erstellung von Modellsegmenten, vergleichbar einem Sägestumpfmodell, und
ggf. Anfertigung eines Inlays/Onlays am oben hergestellten positiven Modell aus Kompositmaterial mit höherer Genauigkeit und in kürzerer Zeit als im Mund des Patienten.

Als Abformmaterial für die Abformnahme zur Herstellung des negativen Abdrucks des abzuformenden Gegenstands werden bevorzugt ein oder mehrere A-Silikone gemäß ISO 4823 (zahnärztliche elastomere Abformmaterialien) verwendet. Diese Materialien sind dünnfließende, mittelfließende, zähfließende oder knetbare additionsvernetzende Silikon-Abformmaterialien, die die Anforderungen nach ISO 4823 hinsichtlich ihrer Verarbeitungs- und Endeigenschaften erfüllen. Es können auch Materialkombinationen von additionsvernetzenden Silikonen gemäß der bekannten Abformtechniken wie z.B. Korrekturabformung oder Doppelmischtechnik zum Einsatz kommen. Insbesondere werden 2-Komponenten additionsvernetzende Silikonmaterialien verwendet, die über hand- oder elektrischbetriebene Misch- und Dosiergeräten, z.B. aus Kartusche oder Folienschlauchbeuteln, ausgetragen und über statische oder dynamische Mischer homogen gemischt werden. Solche Materialien sind zum Beispiel die Handelsprodukte "Monopren transfer, Kettosil oder Panasil tray" der Firma Kettenbach GmbH & Co. KG. Diese Produkte sind im wesentlichen aus folgenden Inhaltsstoffen aufgebaut: Füllstoffe zwischen 20 bis 80 Gew.%, α, ω-Divinylpolydimethylsiloxane zwischen 30 bis 70 Gew.%, Polyhydrogensiloxane zwischen 1 bis 30 Gew.% und Platinkatalysatoren zwischen 0,1% bis 5 Gew.%, sowie Farb- und Hilfsstoffe.

Als additionsvernetzendes Silikon-Modellmaterial zum Abformen des negativen Abdrucks zur Herstellung eines positiven Abdrucks des abzuformenden Gegenstands werden bevorzugt dünnfließende additionsvernetzende Silikone gemäß der veröffentlichten Patentanmeldungsschrift PCT/EP/9601623 verwendet, die nach Aushärten ein E-Modul > 20 MPa und eine Shore D-Härte >35 aufweisen, oder z.B. additionsvernetzende Zahnfleischmaskenmaterialien.

Als Sockelmaterial zur Fixierung der Modellzähne können standfeste mittel- bis zähfließende additionsvernetzende 2-Komponentenmaterialien verwendet werden, die nach Aushärten eine hohe Shore A oder Shore D-Härte aufweisen. Es handelt sich dabei um ein Bissregistriermaterial gemäß PCT/EP/9601623 Als geeignet erweisen sich insbesondere Materialien wie die Handelsprodukte "Futar und Futar D" der Firma Kettenbach GmbH & Co. KG. (Bestandteile und Zusammensetzung siehe Beispiele in der o.g. Patentschrift).

Das erfindungsgemäße Trennmittel und Verfahren wird bevorzugt eingesetzt, wenn der abzuformende Gegenstand ein Zahn, Gebiss oder ein Teil des Gebisses ist, die auch präpariert sein können. Das erfindungsgemäße Trennmittel und Verfahren dient insbesondere zur Herstellung einer Dentalrestauration, wie eines Inlays/Onlays. Das erfindungsgemäße Trennmittel kann in der Zahntechnik und außerhalb der Zahntechnik überall dort eingesetzt werden, wo eine Negativform auf Basis additionsvernetzenden Silikonen mit einem weiteren additionsvernetzendem Silikon abgeformt werden soll, ohne dass ein Haftverbund zwischen diesen beiden additionsvernetzenden Silikonen zustande kommt. Hierzu zählen zum Beispiel Anwendungen bei Modellbau, Halbleitertechnik, Automobilbau oder Elektroindustrie, wo eine Trennwirkung zwischen einem ausgehärteten und einem aushärtenden additionsvernetzenden Material erforderlich ist.

### Beispiel 1

### Herstellung eines erfindungsgemäßen Trennsprays

In einem Glaskolben werden 4 Teile eines Trimethylsiloxy-endgestoppten Polydimethylsiloxans mit einer Viskosität von 50 mPas (bei 20°C) mit 2 Teilen eines anionischen Tensids und 5 Teilen eines durch Äquilibrierung gewonnenen statistischen Gemisches, das zu 50% α-Vinyl, ω-Trimethylsiloxypolydimethylsiloxan, 25% α,ω-Divinylpolydimethylsiloxan und 25% α,ω-Bistrimethylsiloxypolydimethylsiloxan enthält, mit einer Viskosität von 32.000 mPas und einem Vinylgehalt von 0,02 mmol/g homogen gemischt und zusammen mit 89 Teilen einer Propan/Butan-Treibgasmischung in eine Sprühdose überführt und verschlossen.

### Beispiel 2

### Verwendung des erfindungsgemäßen Trennsprays aus Beispiel 1 zur Herstellung eines Kiefermodells

Von einem Kiefer mit präparierten Zähnen wird eine Teilabformung mit einem additionsvernetzenden Silikon Kettosil der Fa. Kettenbach GmbH & Co. KG vorgenommen. Die ausgehärtete negative Abformung wird mit dem erfindungsgemäßen Trennspray aus Beispiel 1 besprüht. Nach kurzer Ablüftzeit wird die mit Trennspray behandelte negative Abformung mit einem additionsvernetzenden dünnfließenden extra harten Modellsilikon der Fa. Kettenbach GmbH & Co. KG ausgegossen ("additionsvernetzende 2-Komponenten Silikonmaterialien" mit hoher Shore D-Härte und hohem E-Modul gemäß der veröffentlicheten Patentanmeldungsschrift PCT/EP96/01623).

Während des Aushärtens des dünnfließenden Modellmaterials wird ein standfestes, mittel- bis zähfließendes, additionsvernetzendes Silikon der Firma Kettenbach GmbH & Co. KG (Futar D) als Sockelmaterial aufgebracht. Dieses Sockelmaterial wird mit Hilfe eines Abformträgers (z.B. Frog der Fa. GC Corp., Japan) aufgebracht, um sich mit dem Modellmaterial zu verbinden und dem positiven Modell in einem Sockel den notwendigen mechanischen Halt zu geben.

Nach Aushärten der beiden additionsvernetzenden Materialien (Modellsilikon und Sockelmaterial) wird die positive Abformung mitsamt dem festverbundenen Sockel aus der negativen Abformung des Kiefers entfernt.

Das Modellmaterial lässt sich hierbei sehr leicht aus der Abformung entnehmen.

Das auf diese Weise hergestellte Modell zeichnet sich durch eine exakte Detailwiedergabe, exakte Kantenschärfe und eine glatte- und blasenfreie Oberfläche aus.

Auf diese Weise erhält der Zahnarzt sehr schnell ein präzises Arbeitsmodell zur stuhlseitigen Herstellung von Dentalrestaurationen, wie z.B. Inlays und Onlays.

### Beispiel 3 (Vergleichsbeispiel, nicht erfindungsgemäß)

### Verwendung eines Trennsprays nach dem Stand der Technik zur Herstellung eines Kiefermodells

Von einem Kiefer mit präparierten Zähnen wird eine Teilabformung mit einem additionsvernetzenden Silikon Kettosil der Fa. Kettenbach GmbH & Co. KG vorgenommen. Die ausgehärtete negative Abformung wird mit einem handelsüblichen Silicontrennspray der Fa. Orbis Dental, Art.-Nr. 43299 [bestehend aus Trimethylsiloxy endgestopptem Polydimethylsiloxan (nicht funktionelle Silicone), Propan-/Butan-Treibgasmischung ca. 85% und Tensid ohne reaktive Gruppen sowie sonstige Hilfs- und Geruchsstoffe.] besprüht.

Nach kurzer Ablüftzeit wird die mit Trennspray behandelte negative Abformung mit einem additionsvernetzenden dünnfließenden extra harten Modellsilikon der Fa. Kettenbach GmbH & Co. KG ausgegossen ("additionsvernetzende 2-Komponenten Silikonmaterialien" mit hoher Shore D-Härte und hohem E-Modul gemäß der veröffentlichten Patentanmeldung PCT/EP96/01623).

Während des Aushärtens des dünnfließenden Modellmaterials wird ein standfestes, mittel- bis zähfließendes, additionsvernetzendes Silikon der Firma Kettenbach GmbH & Co. KG (Futar D) als Sockelmaterial aufgebracht. Dieses Sockelmaterial wird mit Hilfe eines Abformträgers (z.B. Frog der Fa. GC Corp., Japan) aufgebracht, um sich mit dem Modellmaterial zu verbinden und dem positiven Modell in einem Sockel den notwendigen mechanischen Halt zu geben.

Nach Aushärten der beiden additionsvernetzenden Materialien (Modellsilikon und Sockelmaterial) wird die positive Abformung mitsamt den festverbundenen Sockel aus der negativen Abformung des Kiefers entfernt. Hierbei bricht das Modellmaterial an einigen Stellen aufgrund des Haftverbundes zum Abformmaterial ab und verbleibt teilweise in der Negativabformung stecken. Andererseits wird an einigen Stellen Abformmaterial aus der Abformung herausgerissen und haftet am Modellmaterial. Abformung und Modell sind hierdurch unbrauchbar geworden. Eine Herstellung von Dentalrestaurationen ist auf diesem fehlerhaften Modell nicht möglich.

### Beispiel 4 (Vergleichsbeispiel, nicht erfindungsgemäß)

### Versuch der Herstellung eines Kiefermodells ohne Verwendung eines Trennmittels

Von einem Kiefer mit präparierten Zähnen wird eine Teilabformung mit einem additionsvernetzenden Silikon Kettosil der Fa. Kettenbach GmbH & Co. KG vorgenommen.

Die negative Abformung wird ohne Vorbehandlung mit einem Trennmittel mit einem additionsvernetzenden dünnfließenden extra harten Modellsilikon der Fa. Kettenbach GmbH & Co. KG ausgegossen ("additionsvernetzende 2-Komponenten Silikonmaterialien" mit hoher Shore D-Härte und hohem E-Modul gemäß der veröffentlichten Patentanmeldung PCT/EP/9601623).

Während des Aushärtens des dünnfließenden Modellmaterials wird ein standfestes, mittel- bis zähfließendes, additionsvernetzendes Silikon der Fa. Kettenbach GmbH & Co. KG (Futar D) als Sockelmaterial aufgebracht. Dieses Sockelmaterial wird mit Hilfe eines Abformträgers (z.B. Frog der Fa. GC Corp., Japan) aufgebracht, um sich mit dem Modellmaterial zu verbinden und dem positiven Modell in einem Sockel den notwendigen mechanischen Halt zu geben.

Nach Aushärten der beiden additionsvernetzenden Materialien (Modellsilikon und Sockelmaterial) wird versucht die positive Abformung mitsamt dem festverbundenen Sockel aus der negativen Abformung des Kiefers zu entfernen.

Das Modellmaterial ist hierbei jedoch untrennbar mit der negativen Kieferabformung verbunden.

Dieses Beispiel zeigt, dass die Herstellung eines Arbeitsmodells durch Ausgießen einer negativen Form aus additionsvernetzenden Silikonen mit einem additionsvernetzenden Modellsilikon nicht möglich ist.

## Patentansprüche

1. Trennmittel mit einem Treibgas oder Lösungsmittel und einer weiteren Komponente ausgewählt aus der Gruppe umfassend Silikone, Silane und Tenside, die pro Molekül eine einzige ethylenisch ungesättigte Struktureinhelt aufweist.

2. Trennmittel nach Anspruch 1, das als ethylenisch ungesättigte Struktureinheit eine Vinylgruppe, die direkt oder über einen Spacer am Si-Atom gebunden ist, aufweist.

3. Trennmittel nach Anspruch 1 und/oder 2, **dadurch gekennzeichnet, dass** es
Silane, die pro Molekül eine ethylenisch ungesättigte Struktureinheit aufweisen, wie Alkenylalkylsilane, Alkenyl-arylsilane, Alkenylalkylarylsi-lane, Alkenyl(alkylsiloxy)sitane, Alkenyl(arylsiloxy)-silane, Alkenyl(alkylarylsiloxy)silane, wobei die Alkyl- und/oder Arylgruppen auch weitere Substituenten enthalten können; sowie bevorzugt Silane aus der Gruppe Vinyltrimethylsilan, Vinyltriethylsilan, Vinyldiethylmethylsilan, Vinyl-t-butyldimethylsilan, Vinylphenyldimethylsilan, Vinyl(trifluoromethyl)dimethylsilan, Vinyl-(3,3,3-trifluorpropyl)dimethylsilan, Vinyltriphenylsilan und Vinyltris-(trimethylsiloxy)silan,
und/oder Silikonpolymere, die pro Molekül eine ethylenisch ungesättigte Struktureinheit aufweisen, wie α-Alkenyl,-ω-trimethylsiloxy endgestoppte Polydialkylsiloxane, Polydiarylsiloxane und Polyalkylarylsiloxane, wobei die Alkyl- und/oder Arylgruppen gegebenenfalls auch weitere Substituenten enthalten können; sowie insbesondere α-Vinyl-,ω-Trimethylsiloxy-Polydimethylsiloxane,
und/oder zyklische Oligo- oder Polydialkyl-, Diaryl- oder -alkylaryl-Siloxane, die pro Molekül eine ethylenisch ungesättigte Struktureinheit aufweisen, bevorzugt zyklische Vinyloligo- oder Vinylpolydimethylsiloxane wie beispielsweise Vinylheptamethyl-zyklotetrasiloxan,
und/oder feste oder flüssige QM-Harze, die pro Molekül eine ethylenisch ungesättigte Struktureinheit aufweisen, Silandendrimere oder Siloxandendrimere, die pro Molekül eine terminale Alkenylgruppe aufweisen, Tenside, die eine Alkenyl- oder Si-Alkenyl-Struktureinheit enthalten und/oder Silikonpolymere ohne funktionelle Struktureinheiten
umfaßt.

4. Trennmittel nach einem der Ansprüche 1 bis 3, enthaltend Silikonpolymere, zyklische Siloxane oder QM-Harze, die mehr als eine ethylenisch ungesättigte Struktureinheit pro Molekül aufweisen und in Form eines statistischen Gemisches mit Verbindungen ohne ethylenisch ungesättigte Doppelbindungen eingesetzt werden.

5. Trennmittel nach einem der Ansprüche 1 bis 4, **dadurch gekennzeichnet, dass** das Treibgas Propan und/oder Butan ist oder dass das Lösungsmittel ein Aromat, Benzin, Ether, Ester, Alkohol, Keton, Aldehyd, Amin, Carbonsäure, halogeniertes Lösungsmittel, z.B. Chloroform, zyklisches oder lineares Oligo- oder Polysiloxan oder Wasser oder mögliche Kombinationen aus den oben genannten Lösungsmitteln ist.

6. Trennmittel nach einem der Ansprüche 1 bis 5, **dadurch gekennzeichnet, dass** es oberflächenaktive Verbindungen enthält.

7. Trennmittel nach Anspruch 6, **dadurch gekennzeichnet, dass** es anionische, kationische und/oder nicht Ionische Tenside enthält.

8. Trennmittel nach einem der Ansprüche 1 bis 7, **dadurch gekennzeichnet, dass** es Markierungsmittel, insbesondere Metalloxide, Metalle, Titandioxid, Zinkoxid oder Siliciumdioxid, und/oder Farbstoffe, insbesondere lösliche Farbstoffe oder Pigmentfarbstoffe, enthält.

9. Verfahren zur Herstellung eines positiven Abdrucks eines mit Silikonen abgeformten Gegenstandes umfassend folgende Schritte:
Abformnahme eines abzuformenden Gegenstandes mit additionsvernetzenden Silikonen,
Aushärten des additionsvernetzenden Silikons am abzuformenden Gegenstand,
Ablösen der gebildeten negativen Abform des abzuformenden Gegenstandes aus ausgehärtetem additionsvernetzendem Silikon-Polymer,
Besprühen, Bepinseln oder Eintauchen der negativen Abform des abzuformenden Gegenstandes mit einem Trennmittel nach einem der
Ansprüche 1 bis 8, gegebenenfalls gefolgt durch eine visuelle Kontrolle, optisches Abtasten oder Scannen,
Abformen der negativen Abform des abzuformenden Gegenstandes mit dem additionsvernetzendem Silikon und
Entfernen der positiven Abform des abzuformenden Gegenstandes,
ggf. gefolgt von einer weiteren Bearbeitung des positiven Abdrucks.

10. Verfahren zur Herstellung eines .positiven Abdruckes eines Gegenstandes verbunden mit einem Sockel umfassend folgende Schritte:
Abformnahme eines abzuformenden Gegenstandes mit additionsvernetzenden Silikonen,
Aushärten des additionsvernetzenden Silikons am abzuformenden Gegenstand,
Ablösen der gebildeten negativen Abformung des abzuformenden Gegenstandes aus ausgehärtetem additionsvernetzendem Silikon-Polymer,
Besprühen, Bepinseln oder Eintauchen der negativen Abformung des abzuformenden Gegenstandes mit oder in einem Trennmittel nach einem der Ansprüche 1 bis 8, gegebenenfalls gefolgt durch eine visuelle Kontrolle, optisches Abtasten oder Scannen,
Abformen der negativen Abformung des abzuformenden Gegenstandes mit dem additionsvernetzenden Silikon, insbesondere mit extra hartem Modellsilikon,
Aufbringen eines Sockelmaterials, vorzugsweise standfestes mittelbis zähfließendes additionsvernetzendes silikon, während des Aushärtens des additionsvernetzenden Silikonmodellmaterials, um das positive Modell mit einem Sockel zu versehen und den notwendigen mechanischen Halt zu geben,
Aushärten des additionsvernetzendem Silikons (extra hartes Modellsilikon) und des additionsvernetzendem Silikonsockelmaterials,
Entfernen der positiven Abformung mitsamt des fest verbundenen Sockels aus der negativen Abformung des abzuformenden Gegenstandes,
Bearbeitung des positiven Modells zur Erstellung von Modellsegmenten vergleichbar einem Sägestumpfmodell, bevorzugt mit einem Skalpell, einem Messer oder einer Säge,
und gegebenenfalls Anfertigung eines Inlays/Onlays am oben hergestellten positiven Modell aus Kompositmaterial.

11. Verfahren nach Anspruch 9 oder 10, wobei als additionsvernetzende Silikone ein oder mehrere A-Silikone gemäß ISO 4823 (zahnärztliche elastomere Abformmaterialien), die bevorzugt dünnfließend, mittelfließend, zähfließend oder knetbar sind, und/oder 2-Komponenten additionsvernetzende Silikonmaterialien verwendet werden.

12. Verfahren nach einem der Ansprüche 9 bis 11, wobei der abzuformende Gegenstand ein Zahn, Gebiß oder ein Teil des Gebisses ist, die auch präpariert sein können.

13. Verfahren nach einem der Ansprüche 9 bis 12, wobei die negative Abform der Abform des abzuformenden Gegenstandes ein Abbild eines herzustellenden Inlays ist.

14. Verwendung eines Trennmittels nach einem der Ansprüche 1 bis 8 bei einem Verfahren, bei dem eine Trennwirkung zwischen einem ausgehärteten und einem aushärtenden addtionsvernetzenden Material erforderlich ist.

## Claims

1. A separating agent comprising a propellant or solvent and a further component selected from the group comprising silicones, silanes, and surfactants having a single ethylenically unsaturated structural unit per molecule.

2. A separating agent according to claim 1 having as ethylenically unsaturated structural unit a vinyl group, which is bonded to the Si atom either directly or via a spacer.

3. A separating agent according to claim 1 and/or claim 2, **characterized in that** it comprises
silanes having one ethylenically unsaturated structural unit per molecule, such as alkenylalkylsilanes, alkenylarylsilanes, alkenylalkylarylsilanes, alkenyl(alkylsiloxy)silanes, alkenyl(arylsiloxy)silanes, alkenyl(alkylarylsiloxy)-silanes, wherein the alkyl and/or aryl groups can also comprise further substituents; and preferably silanes from the group of vinyltrimethylsilane, vinyltriethylsilane, vinyldiethylmethylsilane, vinyl-t-butyldimethylsilane, vinylphenyldimethylsilane, vinyl(trifluoromethyl)dimethylsilane, vinyl-(3,3,3-trifluoropropyl)dimethylsilane, vinyltriphenylsilane, and vinyltris-(trimethylsiloxy)silane;
and/or silicone polymers having one ethylenically unsaturated structural unit per molecule, such as α-alkenyl,-ω-trimethylsiloxy-terminated polydialkylsiloxanes, polydiarylsiloxanes, and polyalkylarylsiloxanes, wherein the alkyl and/or aryl groups can optionally comprise further substituents; and in particular α-vinyl-,ω-trimethylsiloxypolydimethylsiloxanes;
and/or cyclic oligo- or polydialkyl-, diaryl-, or-alkylarylsiloxanes having one ethylenically unsaturated structural unit per molecule, preferably cyclic vinyloligo- or vinylpolydimethylsiloxanes such as for example vinylheptamethyl-cyclotetrasiloxane;
and/or solid or liquid QM resins having one ethylenically unsaturated structural unit per molecule, silane dendrimers or siloxane dendrimers having a terminal alkenyl group per molecule, surfactants comprising an alkenyl or Si-alkenyl structural unit and/or silicone polymers without functional structural units.

4. A separating agent according to any one of claims 1 to 3 comprising silicone polymers, cyclic siloxanes, or QM resins, which have more than one ethylenically unsaturated structural unit per molecule and are used in the form of a random mixture with compounds without ethylenically unsaturated double bonds.

5. A separating agent according to any one of claims 1 to 4, **characterized in that** the propellant is propane and/or butane, or the solvent is an aromatic compound, benzine, an ether, ester, alcohol, ketone, aldehyde, amine, carboxylic acid, halogenated solvent, for example chloroform, cyclic or linear oligo- or polysiloxane, or water, or possible combinations of the solvents mentioned above.

6. A separating agent according to any one of claims 1 to 5, **characterized in that** it comprises surface-active compounds.

7. A separating agent according to claim 6, **characterized in that** it comprises anionic, cationic and/or nonionic surfactants.

8. A separating agent according to any one of claims 1 to 7, **characterized in that** it comprises matting agents, in particular metal oxides, metals, titanium dioxide, zinc oxide, or silicon dioxide, and/or dyes, in particular soluble dyes or pigment dyestuffs.

9. A method for making a positive impression of an object of which an impression has been taken using silicones, said method comprising the following steps:
taking an impression of an object to be impressed using addition-crosslinking silicones;
curing the addition-crosslinking silicone at the object to be impressed;
removing the negative impression made of the object to be impressed of cured addition-crosslinking silicone polymer;
spraying or painting the negative impression of the object to be impressed with a separating agent according to any one of claims 1 to 8 or immersing the negative impression in said separating agent, optionally followed by visual inspection, or optical scanning;
taking an impression of the negative impression of the object to be impressed using the addition-crosslinking silicone; and
removing the positive impression of the object to be impressed;
optionally followed by additional processing of the positive impression.

10. A method for making a positive impression of an object, which is connected to a base, said method comprising the following steps:
taking an impression of an object to be impressed using addition-crosslinking silicones;
curing the addition-crosslinking silicone at the object to be impressed;
removing the negative impression made of the object to be impressed of cured addition-crosslinking silicone polymer;
spraying or painting the negative impression of the object to be impressed with a separating agent according to any one of claims 1 to 8 or immersing the negative impression in said separating agent, optionally followed by visual inspection, or optical scanning;
taking an impression of the negative impression of the object to be impressed using the addition-crosslinking silicone, in particular using extra hard model silicone;
applying a base material, preferably non-sag medium-bodied to heavy-bodied addition-crosslinking silicone, while the addition-crosslinking silicone model material cures to provide the positive model with a base and to give the necessary mechanical support;
curing the addition-crosslinking silicone (extra hard model silicone) and addition-crosslinking silicone base material;
removing the positive impression together with the firmly attached base from the negative impression of the object to be impressed;
processing the positive model to prepare model segments comparable to a saw model, preferably using a scalpel, knife, or saw;
and optionally preparing an inlay/onlay on the positive model made above of composite material.

11. A method according to claim 9 or claim 10 wherein as addition-crosslinking silicones, one or more A-silicones according to ISO 4823 (dental elastomeric impression materials), which are preferably light-bodied, medium-bodied, heavy-bodied, or putty, and/or two-component addition-crosslinking silicone materials are used.

12. A method according to any one of claims 9 to 11 wherein the object to be impressed is a tooth, a full set of teeth, or a partial set of teeth, which can also be prepared.

13. A method according to any one of claims 9 to 12 wherein the negative impression of the impression of the object to be impressed is an image of an inlay to be prepared.

14. The use of a separating agent according to any one of claims 1 to 8 in a method in which a separating effect between a cured and a curing addition-crosslinking material is required.

## Revendications

1. Agent de démoulage avec un gaz propulseur ou un solvant et un autre composant choisi dans le groupe comprenant les silicones, les silanes et les agents tensioactifs et comportant, par molécule, une seule unité structurelle éthyléniquement insaturée.

2. Agent de démoulage selon la revendication 1, comportant en tant qu'unité structurelle éthyléniquement insaturée un groupe vinyle relié directement ou à travers un espaceur à l'atome Si.

3. Agent de démoulage selon les revendications 1 et/ou 2, **caractérisé en ce qu'**il comprend
des silanes comportant, par molécule, une unité structurelle éthyléniquement insaturée comme les alcénylalkylsilanes, les alcényl-arylsilanes, les alcénylalkylarylsilanes, les alcényl(alkylsiloxy)silanes, les alcényl(arylsiloxy)-silanes, les alcényl(alkylarylsiloxy)silanes, les groupements alkyle et/ou aryle pouvant contenir d'autres substituants ; ainsi que préférentiellement des silanes issus du groupe vinyltriméthylsilane, vinyltriéthylsilane, vinyidiéthylméthylsilane, vinyl-t-butyldiméthylsilane, vinylphényldiméthylsilane, vinyl(trifluorométhyl)diméthylsilane, vinyl-(3,3,3-trifluoropropyl)diméthylsilane, vinyltriphénylsilane et vinyltris-(triméthylsiloxy)silane,
et/ou des polymères de silicone comportant, par molécule, une unité structurelle éthyléniquement insaturée tels que les polydialkylsiloxanes, les polydiarylsiloxanes et les polyalkylarylsiloxanes présentant des groupements terminaux α-alcényl,ω-triméthylsiloxy, les groupements alkyle et/ou aryle pouvant le cas échéant contenir d'autres substituants ; ainsi que notamment des α-vinyl-,ω-triméthylsiloxypolydiméthylsiloxanes,
et/ou des siloxanes cycliques de type oligo- ou polydialkyl, -diaryl- ou -alkylaryl comportant, par molécule, une unité structurelle éthyléniquement insaturée, de préférence des vinyloligo- ou vinylpolydiméthylsiloxanes tels que, par exemple, le vinylheptaméthyl-cyclotétrasiloxane,
et/ou des résines solides ou liquides de type QM comportant, par molécule, une unité structurelle éthyléniquement insaturée, des dendrimères de silanes ou des dendrimères de siloxanes comportant, par molécule, un groupement alcényle terminal, des agents tensioactifs contenant une unité structurelle alcényle ou Si-alcényle et/ou des polymères de silicone sans unités structurelles fonctionnelles.

4. Agent de démoulage selon l'une des revendications 1 à 3, contenant des polymères de silicone, des siloxanes cycliques ou des résines QM comportant, par molécule, plus d'une unité structurelle éthyléniquement insaturée et étant mis en oeuvre sous forme d'un mélange statistique avec des composés sans doubles liaisons éthyléniquement insaturées.

5. Agent de démoulage selon l'une des revendications 1 à 4, **caractérisé en ce que** le gaz propulseur est du propane et/ou du butane et que le solvant et un composé benzénique, une essence, un éther, un ester, un alcool, une cétone, un aldéhyde, une amine, un acide carboxylique, un solvant halogéné, par exemple du chloroforme, un oligo- ou polysiloxane cyclique ou linéaire ou de l'eau ou bien une combinaison des solvants mentionnés ci-dessus.

6. Agent de démoulage selon l'une des revendications 1 à 5, **caractérisé en ce qu'**il contient des composés aux propriétés tensioactives.

7. Agent de démoulage selon la revendication 6, **caractérisé en ce qu'**il contient des agents tensioactifs anioniques, cationiques et/ou non-ioniques.

8. Agent de démoulage selon l'une des revendications 1 à 7, **caractérisé en ce qu'**il contient des agents de marquage, notamment des oxydes de métaux, des métaux, du dioxyde de titane, de l'oxyde de zinc ou du dioxyde de silicium, et/ou des agents de coloration, notamment des colorants solubles ou des pigments.

9. Procédé de réalisation d'une empreinte positive d'un objet dont l'empreinte a été prise avec des silicones, comprenant les étapes suivantes :
prise d'empreinte d'un objet à répliquer avec des silicones à réticulation par addition,
durcissement du silicone à réticulation par addition sur l'objet à répliquer,
retrait de l'empreinte négative de l'objet à répliquer, ladite empreinte étant constituée de polymère durci à base de silicone à réticulation par addition,
application par pulvérisation, au pinceau ou par immersion de l'agent de démoulage selon l'une des revendications 1 à 8 sur l'empreinte négative de l'objet à répliquer, suivie le cas échéant d'un contrôle visuel, d'un balayage optique ou d'un balayage électronique,
prise d'empreinte sur l'empreinte négative de l'objet à répliquer avec le silicone à réticulation par addition, et
retrait de l'empreinte positive de l'objet à répliquer
le cas échéant suivi d'un traitement ultérieur de l'empreinte positive.

10. Procédé de réalisation d'une empreinte positive d'un objet associé à socle, comprenant les étapes suivantes :
prise d'empreinte d'un objet à répliquer avec des silicones à réticulation par addition,
durcissement du silicone à réticulation par addition sur l'objet à répliquer,
retrait de l'empreinte négative de l'objet à répliquer, ladite empreinte étant constituée de polymère durci à base de silicone à réticulation par addition,
application par pulvérisation, au pinceau ou par immersion de l'agent de démoulage selon l'une des revendications 1 à 8 sur l'empreinte négative de l'objet à répliquer, suivie le cas échéant d'un contrôle visuel, d'un balayage optique ou d'un balayage électronique,
prise d'empreinte sur l'empreinte négative de l'objet à répliquer avec le silicone à réticulation par addition, notamment avec un silicone de moulage extra dur,
application d'un matériau de socle, s'agissant de préférence d'un silicone à réticulation par addition présentant une viscosité moyenne à élevée, durant le durcissement du matériau de moulage de type silicone à réticulation par addition afin de pourvoir le modèle positif d'un socle et de lui conférer la tenue mécanique requise,
durcissement du silicone à réticulation par addition (silicone de moulage extra dur) et du matériau de socle de type silicone à réticulation par addition,
retrait de l'empreinte positive et du socle solidement relié à cette dernière de l'empreinte négative de l'objet à répliquer,
travail du modèle positif afin de créer des segments de modèle comparables avec un die, de préférence avec un scalpel, un couteau ou une scie,
et, le cas échéant, réalisation d'un inlay/onlay sur le modèle réalisé ci-dessus en matériau composite.

11. Procédé selon les revendications 9 ou 10, les silicones à réticulation par addition mis en oeuvre étant un ou plusieurs silicones de type A selon ISO 4823 (produits pour empreintes, à base d'élastomères) présentant une viscosité faible, moyenne ou élevée ou étant malaxables et/ou des matériaux à base de silicones à réticulation par addition mettant en oeuvre deux composants.

12. Procédé selon l'une des revendications 9 à 11, l'objet à répliquer étant une dent, un dentier ou une partie d'un dentier qui peuvent également être préparés.

13. Procédé selon l'une des revendications 9 à 12, l'empreinte négative de l'empreinte de l'objet à répliquer donnant une réplique de l'inlay à réaliser.

14. Utilisation d'un agent de démoulage selon l'une des revendications 1 à 8 dans un procédé nécessitant un effet de séparation entre un matériau à réticulation par addition déjà durci et un tel matériau qui est en train de durcir.
